Europäisches Patentamt

European Patent Office

Office européen des brevets

(19)

(11) Publication number: **0 065 342**
**B1**

(12) **EUROPEAN PATENT SPECIFICATION**

(45) Date of publication of patent specification: **20.02.85**

(51) Int. Cl.⁴: **C 07 D 209/08**

(21) Application number: **82200585.6**

(22) Date of filing: **13.05.82**

(54) **Process for the preparation of indole or a methyl-substituted indole, possibly mixed with the hydrogenated derivative.**

(30) Priority: **15.05.81 NL 8102390**

(43) Date of publication of application:
**24.11.82 Bulletin 82/47**

(45) Publication of the grant of the patent:
**20.02.85 Bulletin 85/08**

(84) Designated Contracting States:
**BE CH DE FR GB IT LI NL**

(56) References cited:

**JOURNAL OF ORGANIC CHEMISTRY, vol. 27, no. 10, 10th October 1962, pages 3882-3885, (USA); E.F. GODEFROI et al.: "Angularly arylated decahydroquinolines, hexahydroindolines, and octahydropyrindine"**

(73) Proprietor: **STAMICARBON B.V.**
**Postbus 10**
**NL-6160 MC Geleen (NL)**

(72) Inventor: **van der Stoel, Roland Emile**
**Schout Boutenstraat 27**
**NL-6121 HC Buchten (NL)**
Inventor: **Janssen, Petrus Hubert Joseph**
**Mauritspark 32**
**NL-6163 HN Geleen (NL)**
Inventor: **van de Moesdijk, Cornelis Gerardus Maria**
**Drossaert Saldenstraat 7**
**NL-6181 ER Elsloo (NL)**

(74) Representative: **Hoogstraten, Willem Cornelis Roeland et al**
**OCTROOIBUREAU DSM Postbus 9**
**NL-6160 MA Geleen (NL)**

Courier Press, Leamington Spa, England.

## Description

The invention relates to a process for the preparation of indole, in which a substituent methyl group may be bound to one or more of the C atoms in the position 3, 4, 5, 6 and 7, the indole being possibly mixed with the hydrogenated derivative, for instance tetrahydroindole.

Indole and homologues, which are used in, for instance, the fragrances industry, are commercially recovered from coal tar. However, as a result of the very low concentration in coal tar of the compounds concerned (approximately 0,2% by weight) this recovery is very expensive. Up to now, a suitable method for the synthetic preparation of indole and said methyl-substituted indole compounds has not been known. The invention now provides such a method.

The process according to the invention for the preparation of indole, in which a substituent methyl group may be bound to one or more of the C atoms in the positions 3, 4, 5, 6 and 7, the indole being possibly mixed with the hydrogenated derivative, is characterized in that 2-(cyanomethyl)-cyclohexanone, in which an H atom of one or more of the $CH_2$ groups may have been replaced by a methyl group, is contacted, in the gas phase and in the presence of hydrogen, with a dehydrogenation catalyst containing a metal from the 8th group or 1st subgroup of the periodic system of elements according to Mendeleev or a compound of such a metal, to form a reaction mixture containing indole or the pertaining substituted indole and possibly, the hydrogenated derivative.

It is known to cyclize 2-(cyanomethyl)-cyclohexanone (2-oxocyclohexaneacetonitrile) (Journal of Organic Chemistry 27 3882—3885, 1962). However, this concerns a cyclization in the liquid phase with the very costly $LiAlH_4$, in which no indole but only hexahydroindole is formed, the yield being 25%. Also known (Chemische Berichte, 95, pp. 307—318, 1962) is the hydrogenation of cyclohexanone-(2)-1-cyanacetic ester, in which also only hydrogenated product is obtained.

Preferably, a dehydrogenation catalyst is used which contains a metal, or a metal compound, e.g. an oxide, of the group platinum, palladium and rhodium. As a support, materials such as, for example, activated carbon, graphite, silica, zinc oxide, aluminium oxide, magnesium oxide and mixture thereof can be used. The amount of supporting material is for example chosen in such a way that the amount of catalyst is 0.01—10% by weight (calculated as metal relative to the total amount of catalyst material, including support). Aluminium oxide is preferably used as supporting material. If desired, a promoter can be added to the catalyst. By preference, this promoter is an alkali metal or an alkali metal compound, for example an alkali metal oxide, and is used in an amount which is normal for dehydrogenation catalysts, for example 0.01—2% by weight (calculated as alkali metal relative to the total amount of catalyst material, including the support).

The dehydrogenation catalysts used in the process of the invention are known as such, and can for instance be prepared (according to method disclosed in Preparation of catalysts II by G. H. van den Berg and H. Th. Rynten, page 265, Elsevier Amsterdam 1979) by treating the supporting material with a salt of the metal concerned, followed by calcination and reduction.

The process according to the invention can be carried out at various temperatures, for instance at a temperature of 175—350°C. By preference a temperature between 200 and 310°C is applied.

The process according to the invention can be carried out in the ways known in themselves for carrying out catalytic gas-phase reactions, for instance by leading the gaseous starting product, optionally diluted with an inert gas such as nitrogen, together with hydrogen over the catalyst in the form of a fixed bed, applying a space velocity of between, for instance, 0.03 and 2 g starting product per ml catalyst (compacted volume) per hour.

The process according to the invention can be carried out with different amounts of hydrogen, for instance an amount of 1—50 moles of hydrogen per mole starting product. Application of more than 50 moles of hydrogen per mole starting product is also possible, but this does not result in any advantage.

The starting product can be obtained by reaction of cyclohexanone, or the pertaining substituted cyclohexanone, with chloroacetonitrile or 2-chloropropionitrile in the way described in the above-mentioned publication in the Journal of Organic Chemistry.

By cooling the gaseous reaction mixture obtained in the process according to the invention a separation can be achieved into a condensate and hydrogen-containing gas which can be recycled. The condensate obtained can for instance be separated by fractional distillation.

In the following examples the invention will be elucidated.

Example I

Through a vertical tubular reactor (17 mm diameter, 400 mm length) in which there is a catalyst zone of 10 ml (compacted volume), a gaseous mixture of 2-oxocyclohexaneacetonitrile and hydrogen (30 moles of hydrogen per mole of oxonitrile) is passed from top to bottom, for 4 hours.

The catalyst zone is bounded at the bottom by a zone of 5 ml and at the top by a zone of 100 ml inert ceramic material. As catalyst palladium and sodium on $\gamma$-aluminium oxide (0,5 wt. % Pd and 0.4 wt. % Na) is applied. Per ml (compacted volume) of catalyst 0.15 g 2-oxocyclohexaneacetonitrile is led through per hour.

The temperature of the catalyst and the inert material is kept at 300°C by means of a heating jacket round the reactor. After 3 hours the composition of the reaction mixture obtained is determined by passing the mixture for 1 hour through a small vessel which has been cooled to 0°C and by analyzing the condensed product thus obtained gas-chromatographically.

From this analysis and the weight of the amount of 2-oxocyclohexaneacetonitrile which has been passed through in the period of 1 hour, the conversion of the oxonitrile and the yields of indole, tetrahydroindole and hexahydroindole can be calculated.

Conversion is understood to mean the amount of oxonitrile which has been converted (amount of oxonitrile passed through minus amount of oxonitrile in the condensed product) expressed as a percentage of the amount of oxonitrile passed through. The yields of indole, tetrahydroindole and hexahydroindole, respectively, are understood to mean the amounts of indole, tetrahydroindole and hexahydroindole, respectively, in the condensed product, expressed as a percentage of the amounts of indole, tetrahydroindole and hexahydroindole, respectively, which can theoretically be formed from the converted amount of oxonitrile.

The conversion of the oxonitrile amounts to 75% and the indole yield to 27%.

Example II

In the way described in Example 1, a mixture of 2-oxocyclohexaneacetonitrile and hydrogen is passed over a catalyst consisting of platinum and $\gamma$-aluminium oxide (0.5 wt. % platinum). The oxonitrile conversion is 69%, and the yield of indole 11%, of tetrahydroindole 12% and of hexahydroindole 1%.

Example III

In the way described in Example I, but differing in that the temperature of the catalyst and the inert material is kept at 240°C, a mixture of 2-oxocyclohexaneacetonitrile and hydrogen is passed over a catalyst consisting of platinum on $\gamma$-aluminium oxide (0.5 wt. % Pt). The oxonitrile conversion is 100%, and the yield of indole 18%, of tetrahydroindole 19% and of hexahydroindole 1%.

Example IV

In the way described in Example III, a mixture of 2-oxocyclohexaneacetonitrile and hydrogen is passed over a catalyst consisting of rhodium on $\gamma$-aluminium oxide (0.5 wt. % Rh). The conversion of the oxonitrile is 99%, the yield of indole 9%, of tetrahydroindole 26% and of hexahydroindole 5%.

Example V

In the way described in Example IV, for 7 hours a mixture of 2-oxocyclohexaneacetonitrile and hydrogen is passed over a catalyst consisting of palladium and sodium on $\gamma$-aluminiumoxide (0.5 wt. % Pd and 0.4 wt. %

Na). After 6 hours the composition of the reaction mixture obtained is determined in the way described in Example I. The conversion of the oxonitrile amounts to 93%, the indole yield to 55%, the tetrahydroindole yield to 12% and the hexahydroindole yield to 3%.

Example VI

Example V is repeated with 2-(2-oxocyclohexane)propionitrile as starting compound. The conversion of the oxonitrile amounts to 85%.

The 3-methylindole (skatole) yield amounts to 45%, the 3-methyltetrahydroindole yield to 10% and the 3-methylhexahydroindole yield to 2%.

Example VII

Example V is repeated with (4-methyl-2-oxocyclohexane)acetonitrile as starting compound. The oxonitrile conversion amounts to 90%, the 6-methylindole yield is 50%, the 6-methyltetrahydroindole yield 9% and the 6-methylhexahydroindole yield 1%.

**Claims**

1. Process for the preparation of indole, in which a substituent methyl group may be bound to one or more of the C atoms in the positions 3, 4, 5, 6 and 7, the indole being possibly mixed with the hydrogenated derivative, characterized in that 2-(cyanomethyl)cyclohexanone, in which an H atom of one or more of the $CH_2$ groups may have been replaced by a methyl group, is contacted, in the gas phase and in the presence of hydrogen, with a dehydrogenation catalyst, containing a metal from the 8th group or 1st sub-group of the periodic system of elements according to Mendeleev, or a compound of such a metal, to form a reaction mixture containing indole or the pertaining substituted indole and possibly the hydrogenated derivative.

2. Process according to claim 1, characterized in that a catalyst is applied which contains a metal or a compound of a metal from the group consisting of platinum, palladium and rhodium.

3. Process according to claim 1 or 2, characterized in that the catalyst is applied on aluminium oxide as supporting material.

4. Process according to any of the claims 1—3, characterized in that also an alkali metal or alkalimetal compound is used as a promoter.

5. Process according to any of the claims 1—4, characterized in that the contact with the catalyst is established at a temperature between 200 and 310°C.

6. Process according to any of the claims 1—5, characterized in that per mole of starting product 1—50 moles of hydrogen are used.

**Patentansprüche**

1. Verfahren zur Herstellung von Indol, in welchem eine substituierende Methylgruppe an eines oder mehrere der C-Atome in den 3-, 4-, 5-, 6- und 7-Stellungen gebunden sein kann und das Indol gegebenenfalls im Gemisch mit dem hydrierten Derivat vorliegt, dadurch gekennzeichnet, daß 2-(Cyanomethyl)cyclohexanon, in welchem ein H-Atom einer oder mehrerer der $CH_2$-Gruppen durch eine Methylgruppe ersetzt worden sein kann, in der Gasphase und in Gegenwart von Wasserstoff mit einem Dehydrierungskatalysator, der ein Metall der Gruppe VIII oder 1. Untergruppe des Periodensystems der Elemente nach Mendeleev oder eine Verbindung eines solchen Metalles enthält, in Berührung gebracht wird, um ein Reaktionsgemisch, enthaltend Indol oder das entsprechende substituierte Indol und gegebenenfalls das hydrierte Derivat, zu bilden.

2. Verfahren nach Anspruch 1, dadurch gekennzeichnet, daß ein Katalysator enthaltend ein Metall oder eine Verbindung eines Metalles der Gruppe bestehend aus Platin, Palladium und Rhodium eingesetzt wird.

3. Verfahren nach Anspruch 1 oder 2, dadurch gekennzeichnet, daß der Katalysator auf Tonerde als Trägermaterial eingesetzt wird.

4. Verfahren nach einem der Ansprüche 1—3, dadurch gekennzeichnet, daß ein Alkalimetall oder eine Alkalimetallverbindung als Promotor eingesetzt wird.

5. Verfahren nach einem der Ansprüche 1—4, dadurch gekennzeichnet, daß der Katalysator bei einer Temperatur von 200 bis 310°C eingesetzt wird.

6. Verfahren nach einem der Ansprüche 1—5, dadurch gekennzeichnet, daß pro Mol Ausgangsprodukt 1—50 Mole Wasserstoff eingesetzt werden.

**Revendications**

1. Procédé de préparation d'indole dans lequel un groupe substituant méthyle peut être lié à un ou plusieurs des atomes de carbone dans les positions 3, 4, 5, 6 et 7, l'indole étant éventuellement mélangé avec un dérivé hydrogéné, caractérisé en ce que la 2-(cyanométhyl)cyclohexanone, dans laquelle un atome d'hydrogène d'un ou de plusieurs groupes $CH_2$ peut avoir été remplacé par un groupe méthyle, est mise en contact, en phase gazeuse et en présence d'hydrogène, avec un catalyseur de déshydrogénation contenant un métal appartenant au 8ème groupe ou au 1er sous-groupe du système périodique des éléments de Mendéléiev, ou un composé d'un tel métal, pour former un mélange réactionnel contenant l'indole ou l'indole substitué correspondant, et, éventuellement, le dérivé hydrogéné.

2. Procédé selon la revendication 1, caractérisé en ce qu'on utilise un catalyseur contenant un métal, ou un composé d'un métal appartenant au groupe du platine, du palladium et du rhodium.

3. Procédé selon l'une des revendications 1 et 2, caractérisé en ce qu'on applique le catalyseur sur de l'oxyde d'aluminium, comme support.

4. Procédé selon l'une quelconque des revendications 1 à 3, caractérisé en ce qu'on utilise aussi un métal alcalin, ou un composé de métal alcalin, comme activeur.

5. Procédé selon l'une quelconque des revendications 1 à 4, caractérisé en ce qu'on établit le contact avec le catalyseur à une température comprise entre 200 et 310°C.

6. Procédé selon l'une quelconque des revendications 1 à 5, caractérisé en ce qu'on utilise 1 à 50 moles d'hydrogène par mole de produit de départ.